# FASCICULE DE BREVET EUROPEEN

(11) **EP 3 175 224 B1**
(45) Date de publication et mention de la délivrance du brevet: **14.03.2018**
(21) Numéro de dépôt: 15756961.7
(22) Date de dépôt: 02.07.2015
(51) Int. Cl.: G01N 21/3563, G01N 21/359, G01N 21/35, G01N 21/31

(54) **PROCÉDÉ ET DISPOSITIF DE DÉTECTION ET DE QUANTIFICATION DE LA FIBROSE**
VERFAHREN UND VORRICHTUNG ZUM NACHWEIS UND ZUR QUANTIFIZIERUNG VON FIBROSE
METHOD AND DEVICE FOR DETECTING AND QUANTIFYING FIBROSIS

(30) Priorité: 29.07.2014 FR 1457323
(43) Date de publication de la demande: 07.06.2017
(73) Titulaire: Université De Reims Champagne-Ardenne, 51100 Reims (FR)
(72) Inventeur: VUIBLET, Vincent, F-51140 Chenay (FR); PIOT, Olivier, F-51350 Cormontreuil (FR); RIEU, Philippe, F-51220 Merfy (FR); FERE, Michael, F-51100 Reims (FR); GOBINET, Cyril, F-02840 Athies-Sous-Laon (FR)
(74) Mandataire: Cabinet Bleger-Rhein-Poupon
(86) Numéro de dépôt international: PCT/FR2015/051826
(87) Numéro de publication internationale: WO 2016/016531

(56) Documents cités:
- WO-A2-02/46722
- US-A- 6 146 897
- JAYAKRUPAKAR NALLALA ET AL: "Infrared spectral histopathology for cancer diagnosis: a novel approach for automated pattern recognition of colon adenocarcinoma", THE ANALYST, vol. 139, no. 16, 12 juin 2014 (2014-06-12), pages 4005-4015, XP055180381, ISSN: 0003-2654, DOI: 10.1039/C3AN01022H
- KWAK JIN TAE ET AL: "Multimodal microscopy for automated histologic analysis of prostate cancer", BMC CANCER, BIOMED CENTRAL, LONDON, GB, vol. 11, no. 1, 9 février 2011 (2011-02-09), page 62, XP021087273, ISSN: 1471-2407, DOI: 10.1186/1471-2407-11-62
- NALLALA JAYAKRUPAKAR ET AL: "Infrared spectral imaging as a novel approach for histopathological recognition in colon cancer diagnosis", JOURNAL OF BIOMEDICAL OPTICS, S P I E - INTERNATIONAL SOCIETY FOR OPTICAL ENGINEERING, US, vol. 17, no. 11, 1 novembre 2012 (2012-11-01), page 116013, XP060023709, ISSN: 1083-3668, DOI: 10.1117/1.JBO.17.11.116013 [extrait le 2012-11-01]

## Description

La présente invention entre dans le domaine médical, pour le diagnostic et le suivi de l'évolution de la fibrose tissulaire.

A cet effet, l'invention concerne particulièrement un système de détection et de quantification de la fibrose tissulaire.

La fibrose tissulaire désigne l'accumulation de matrice extracellulaire au sein du tissu conjonctif. La fibrose intervient suite à une inflammation ou à une lésion dudit tissu. Ce dernier ne va pas se régénérer naturellement et correctement.

La fibrose est un processus d'accumulation de collagène qui est synthétisé par des cellules dans le tissu conjonctif. Le collagène est une famille de protéine physiologiquement présente dans l'organisme pour notamment conférer au tissu ses propriétés de résistance mécanique à l'étirement. Cependant, au sein du parenchyme d'un organe, l'accumulation excessive de collagène, donc de tissu conjonctif résultant de la fibrose, entraine une perte progressive, souvent inéluctable, de la fonction de l'organe.

De ce fait, il existe plusieurs types de fibroses altérant la fonction des organes et entraînant diverses pathologies.

On parle de fibrose pulmonaire pour désigner une altération du tissu conjonctif pulmonaire, dont les alvéoles se retrouvent alors enserrées et étouffées par des fibres de collagène.

On parle de fibrose hépatique pour un tissu conjonctif fibreux se développant là où les cellules hépatiques ont été détruites. Quand la fibrose hépatique s'étend, elle peut aboutir au développement d'une cirrhose.

On parle également de fibrose rénale qui correspond à une accumulation excessive de matrice extracellulaire dans le parenchyme rénal.

En d'autres termes, il existe une corrélation étroite entre le degré de fibrose d'un organe et sa fonction. De manière générale, plus l'accumulation de collagène est excessive dans les tissus conjonctifs, plus la perte de fonction de l'organe est importante.

En conséquence, quantifier spécifiquement la fibrose tissulaire est nécessaire afin de définir un pronostic vital ou fonctionnel et pour suivre l'évolution d'une fibrose existante,

De manière connue, on réalise la quantification de la fibrose d'un tissu sur lames colorées. Un anatomopathologiste va établir un score en fonction de la surface relative occupée par la fibrose au sein de la biopsie.

On entend par « biopsie » le fait de prélever une toute petite partie d'un organe ou d'un tissu afin d'effectuer des examens médicaux. Cette technique d'analyse sur lames colorées a pour désavantage d'être subjective car elle est réalisée par l'oeil humain. Ce moyen de quantification de la fibrose présente donc une mauvaise reproductibilité intra-utilisateur et inter-utilisateurs, et par conséquent cette quantification est peu représentative et manque de fiabilité.

Pour pallier cette limitation de la quantification subjective sur lames colorées, il est également connu de faire une quantification colorimétrique semi automatisée. Cette technique consiste en une analyse semi automatisée et informatisée basée sur une segmentation automatique des couleurs présentes sur les lames colorées au trichrome de Masson. La technique consiste à fixer les biopsies tissulaires, les déshydrater, puis à les inclure dans de la paraffine. Des coupes de paraffine de quatre à dix micromètres (*µ*m) sont étalées sur des supports de type lames, notamment compatibles avec la spectroscopie Infrarouge à Transformée de Fourier (« IRTF ») puis colorée au Trichrome de Masson. Le Trichrome de Masson colore en vert les fibres de collagène.

Après coloration au Trichrome de Masson, les lames sont scannées puis, à partir des images obtenues, un opérateur sélectionne les zones de confusion apparaissant en vert, c'est-à-dire les zones où le collagène est physiologiquement présent. L'image après sélection subit ensuite un traitement informatique basé sur un algorithme fondé sur la segmentation de couleurs, identifiant sur trois canaux les couleurs rouge, vert et bleu. Le vert correspondant au tissu conjonctif avec un excès de collagène et donc à la fibrose.

Cependant, cette quantification colorimétrique semi automatisée présente comme inconvénient de nécessiter une étape manuelle pour éliminer les zones de confusion, introduisant une part de subjectivité et un temps de traitement supplémentaire.

En outre, à ce jour, cette technique n'a été validée qu'au niveau du tissu rénal. Par ailleurs, cette technique surévalue systématiquement la fibrose rénale. En effet, les membranes basales tubulaires rénales sont constituées de collagène, donc colorées en vert et considérées comme de la fibrose. Ce défaut est d'autant plus important que les tubes sont jointifs et donc que la fibrose interstitielle est minime. Ainsi, sur une biopsie exempte de fibrose, une quantification 15 à 20 % de fibrose est obtenue, témoignant d'une surestimation de ladite quantification.

La quantification colorimétrique semi-automatique ne permet pas de différencier les différents types de collagènes au sein d'un même tissu, car elle est basée sur une coloration par le vert colorant tous les collagènes. Bien que reproductible pour la fibrose tissulaire rénale, la quantification colorimétrique semi automatisée manque donc de représentativité.

Une technique équivalente utilise la spectroscopie Roman. Il est également connu pour quantifier la fibrose, notamment au niveau hépatique, la technique de Génération de Seconde Harmonique (SHG pour « Second-Harmonie Génération ») qui permet de réaliser une quantification semi automatisée du collagène de type I présent dans les tissus. La technique SHG se base sur l'émission spécifique du collagène de type I après excitation par un microscope bi-photonique. Après fixation des biopsies tissulaires, déshydratation et inclusion dans de la paraffine, des coupes de 4 à 6 *µ*m sont réalisée puis étalées sur supports compatibles avec la FTIR, sans aucune coloration. Les biopsies tissulaires présentes sur les lames sont segmentées en plusieurs fragments et sont ensuite concaténées par le système. Après excitation, le collagène de type I va émettre un signal SHG. Sur chaque coupe, le signal SHG est détecté. Le signal SHG tissulaire est quantifié via un traitement numérique semi automatisé. Un opérateur devra définir pour chaque image ou groupe d'image les valeurs seuils de bruit et de positivité du signal SHG. Un algorithme de quantification traite ainsi chaque image. L'algorithme va calculer comme résultat la surface relative occupée par le signal SHG, spécifique du collagène de type I, pour chaque biopsie selon les seuils définis par l'opérateur. Il en résulte que cette technique présente comme inconvénient une intervention de l'utilisateur qui doit déterminer subjectivement un seuil de positivité du signal SHG.

De plus, l'imagerie SHG a été décrite uniquement pour la fibrose hépatique et pulmonaire.

En outre, la technique SHG présente comme autre désavantage de ne pas distinguer la fibrose des structures histologiques comportant du collagène de type I physiologiquement comme les tissus conjonctifs normaux. Cette technique SHG permet alors la détection de l'ensemble du collagène de type I présent dans le tissu.

WO02/46722-A2 divulgue un procédé de diagnostic de la présence de pathologie dans un échantillon biologique en étudiant des données spectrales d'absorbance infrarouge. La présente invention a pour but de pallier les inconvénients des méthodes précédemment mentionnées, en proposant un système de détection de la fibrose qui soit capable de quantifier et de suivre l'évolution d'une fibrose tissulaire de façon reproductible, automatique, et objective en s'affranchissant de l'intervention de l'opérateur. Ce système permet de distinguer les différentes structures histologiques collagèniques c'est à dire le tissu conjonctif impliqué dans la fibrose tissulaire pathologique du tissu conjonctif présent physiologiquement.

Un tel système comporte tout d'abord un procédé de détection et de quantification de la fibrose dans lequel au moins :
- on positionne sur un support un échantillon tissulaire fixé non coloré ;
- on effectue une acquisition infrarouge par balayage de points dudit échantillon ;
- on convertit le spectre infrarouge rendu par chaque point acquis en au moins une image spectrale ;
- on effectue un traitement numérique de ladite image spectrale par discrimination entre au moins le collagène physiologique et le collagène pathologique issu de la fibrose en attribuant à chaque point une classe spectrale définie par un algorithme classant les spectres selon leur similitudes spectrale ;
- puis on attribue à chaque classe un état par comparaison statistique avec un modèle numérique de spectres ;
- on quantifie la surface relative occupée par les points les points de chaque état et on enregistre le résultat.

Selon d'autres caractéristiques additionnelles, non limitatives:
- ladite acquisition infrarouge s'effectua par spectroscopie infrarouge à transformation de Fourrier.
- ledit traitement comprend une étape préalable de prétraitement, consistant en l'élimination spectrale des bruits de fond provenant du support et du détecteur et en la neutralisation des interférences de la paraffine.
- lesdites classes sont au nombre de quatre, dont les états sont nommés : normal, fibrose, collagène non fibreux, inflammation.
- ladite discrimination s'effectue par comparaison de la valeur du spectre de chaque point avec des plages de valeurs de spectres dudit modèle,
- on quantifie statistiquement les points d'une même classe sous la forme d'un pourcentage surfacique de la surface totale dudit échantillon.
- on combine ledit résultat audit modèle avant de réitérer ledit procédé avec un autre échantillon.

De plus, ledit système comprend aussi un dispositif de détection et de quantification de la fibrose, permettant la mise en oeuvre du procédé selon l'invention. Un tel dispositif comprend au moins :
- des moyens de réception d'un support d'un échantillon tissulaire ;
- des moyens d' acquisition par spectroscopie infrarouge à transformation de Fournier positionnés en vis-à-vis desdits moyens de réception, lesdits moyens d'acquisition convertissant le spectre infrarouge rendu par chaque point acquis en au moins une image spectrale ;
- des moyens de traitement numérique de ladite image spectrale par discrimination entre au moins le collagène physiologique et le collagène pathologique en attribuant à chaque point une classe spectrale par comparaison statistique avec un modèle numérique de spectres dont est pourvu lesdits moyens de traitement, lesdits moyens de traitement quantifiant les points possédant une classe spectrale similaire ;
- des moyens d'enregistrement numérique du résultat desdits moyens de traitement.

Selon d'autres caractéristiques additionnelles, non limitatives:
- lesdits moyens de réception sont motorisés et que ledit dispositif comprend des moyens de contrôle des déplacements desdits moyens de réception, de manière à effectuer un balayage relatif dudit support par lesdits moyens d'acquisition infrarouge,

D'autres caractéristiques et avantages de l'invention ressortiront de la description détaillée qui va suivre des modes de réalisation non limitatifs de l'invention, en référence à la figure annexée, représentant schématiquement l'architecture du système de détection et de quantification de la fibrose d'un échantillon.

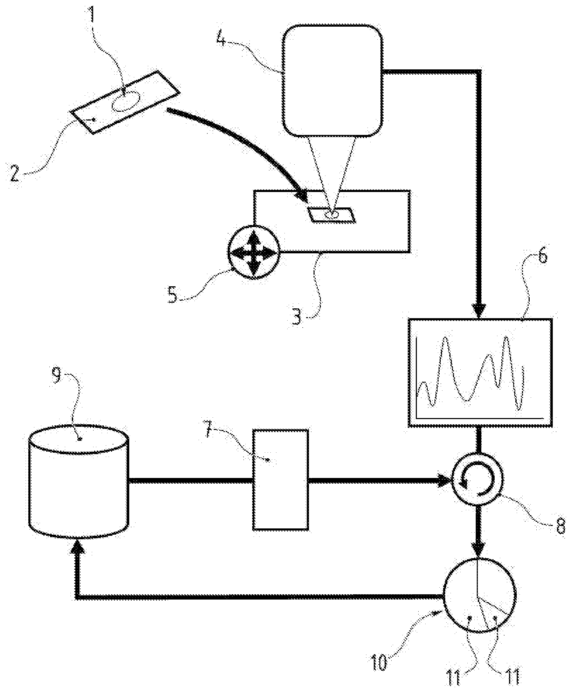

La présente invention concerne un système de détection et de quantification de la fibrose au sein d'un échantillon, illustré sur la figure 1.

Ledit échantillon 1 consiste en un échantillon tissulaire. Tout d'abord, l'échantillon 1 est fixé pour qu'il conserve un état le plus proche possible de l'état in vivo. La fixation de l'échantillon peut être chimique, par exemple avec du formol, de l'AFA, ou du Liquide de Bouin, ou peut être physique, par exemple par congélation ou lyophilisation. L'échantillon 1 fixé est ensuite déshydraté. La déshydratation est effectuée par des moyens connus de l'homme du métier, par exemple à l'aide d'un automate ou par exemple dans des bains d'alcool croissant et en utilisant des solvants organiques intermédiaires tel que le xylène et le toluène.

Ledit échantillon 1 est inclut dans de la paraffine ou de la résine afin d'obtenir un bloc dur. L'inclusion consiste en une imprégnation de l'échantillon dans de la paraffine en surfusion, puis il y a une polymérisation à froid. Après inclusion de l'échantillon, une coupe de l'échantillon inclut est réalisée à l'aide des techniques de microtomie connue. Une telle coupe de l'échantillon peut présenter différentes épaisseurs, en particulier une épaisseur de plusieurs dizaines de micromètres (*µ*m), notamment de 10 à 20 µm.

Cette coupe comprenant l'échantillon 1 est positionnée sur un support 2, de préférence ce dernier consiste en une lame transparente aux rayonnements moyens infrarouges du spectre électromagnétique. La coupe comprenant l'échantillon 1 est étalée sur ledit support 2. Aucune autre préparation supplémentaire n'est nécessaire. A ce titre, ledit échantillon 1 est non coloré.

En dehors de cette opération de préparation, ledit système permet d'automatiser la détection de la fibrose tissulaire sur un échantillon 1 et la quantification de la fibrose tissulaire par rapport aux autres tissus dudit échantillon 1, s'affranchissant de toute intervention humaine, offrant ainsi une reproductibilité accrue et des résultats objectifs.

Pour ce faire, le système selon l'invention fait intervenir une acquisition infrarouge de l'échantillon 1, puis un traitement informatique spécifique des informations spectrales issues de ladite acquisition.

Concernant l'acquisition infrarouge, elle s'effectue par balayage de points dudit échantillon 1.

Pour ce faire, le support 2 est placé au niveau de moyens de réception 3 tels qu'une platine motorisée. Ces derniers permettent de maintenir en place ledit support 2 au cours de l'opération d'acquisition.

Cette acquisition est réalisée par des moyens d'acquisition 4 par spectroscopie infrarouge positionnés en vis-à-vis desdits moyens de réception 3.

Préférentiellement, l'invention fait intervenir une spectroscopie infrarouge à transformation de Fournier. Elle associe une excitation polychromatique à un micro-spectromètre infrarouge pour la détection de l'absorption moyen-infrarouge par l'échantillon 1. De façon subsidiaire, une réfrigération continue du détecteur peut être envisagée, afin de limiter le bruit de mesure.

On notera alors que ledit support 2 est adapté à la spectroscopie par infrarouge, en particulier à transformation de Fournier. En d'autres termes, le support 2 est constitué d'un matériau exempt de signal parasite en infra-rouge, ce matériau est par exemple du Fluorure de Calcium ou ZnSe pour une acquisition en transmission. Des lames de verre métallisées par argentation peuvent également être utilisées pour une acquisition en mode réflexion.

Cette acquisition s'effectue par balayage de points dudit échantillon 1. Un tel balayage peut provenir du déplacement des moyens d'acquisition 4 par rapport aux moyens de réception 3. Préférentiellement, ce sont lesdits moyens de réception 3 qui sont prévus mobiles, tandis que les moyens d'acquisition 4 sont fixes. En d'autres termes, il s'agit d'un balayage relatif dudit support 2 par lesdits moyens d'acquisition 4 infrarouge. Pour ce faire, l'invention intègre des moyens 5 de contrôle des déplacements desdits moyens de réception 3.

En outre, ces moyens de contrôle 5 permet aussi de gérer automatiquement les paramètres d'acquisition des moyens 4.

Ainsi, les moyens de contrôle 5 permettent d'automatiser et de rendre autonome l'étape d'acquisition de l'intégralité de l'échantillon 1.

Lors de l'étape d'acquisition, chaque point rend un spectre infrarouge. Dès lors, on convertit le spectre infrarouge rendu par chaque point acquis en au moins une image spectrale 6.

Ce sont lesdits moyens d'acquisition 4 qui convertissent le spectre infrarouge rendu par chaque point acquis en ladite image spectrale 6.

A ce titre, ladite image spectrale est une représentation numérique de tous les points acquis. Les points sont repérés par leurs coordonnées dans l'espace selon deux dimensions. Chaque point constitue un pixel de ladite image 6.

Afin de quantifier la fibrose, on effectue un traitement numérique de ladite image spectrale 6. Ce traitement s'effectue par des moyens de traitement 8 adaptés. Le traitement consiste en une discrimination entre au moins le collagène physiologique et le collagène pathologique en attribuant à chaque point une classe spectrale par comparaison avec un modèle numérique de spectres 7.

Un tel modèle consiste en l'acquisition d'un large panel dit « d'entraînement » de biopsies rénales avec différents degrés de fibrose, pour lesquels l'attribution de chaque classe a été réalisée par un anatomopathologiste expert en pathologie rénale, puis ce panel a subit une validation interne sur un second panel de biopsies. Un contrôle des résultats du modèle a été effectué puis une validation externe sur un troisième panel de biopsies analysées en aveugle afin de renforcer la robustesse de ce modèle.

Ainsi, à partir des valeurs des spectres mesurés des points de l'échantillon, il est possible de différencier les tissus présentant une fibrose des autres tissus.

En particulier lesdites classes peuvent être au nombre d'au moins deux, mais préférentiellement quatre : collagène physiologique, collagène issue de la fibrose, inflammation et parenchyme « normal » (non collagènique, non fibreux et non inflammatoire). D'autres classes peuvent éventuellement être ajoutées.

Préalablement, le traitement comprend une étape automatisée de prétraitement des spectres, consistant en l'élimination des bruits de fond provenant du support 2 et du détecteur et en la neutralisation des interférences spectrale de la paraffine. Ce prétraitement consiste donc à améliorer la qualité de l'image 6, en éliminant également les spectres de rapport signal/bruit insuffisant. Ledit prétraitement est effectué automatiquement, par un algorithme dédié.

Ensuite, on quantifie statistiquement les points possédant une classe spectrale similaire et on enregistre le résultat 10. Ce sont les moyens de traitement 8 qui assurent automatiquement ces regroupements statistiquement multivariés par classe. Des moyens 9 d'enregistrement numérique assurent ensuite la sauvegarde du résultat 10 issu desdits moyens de traitement 8.

On entend par « similaire » des points ayant reçu en attribution une classe identique ou bien des classes équivalentes. Dans ce dernier cas, plusieurs classes peuvent être regroupées en une seule et même classe globale, de taille plus importante.

En particulier, on quantifie statistiquement les points d'une même classe sous la forme d'un pourcentage surfacique 11 de la surface totale dudit échantillon 1. En d'autres termes, on calcule le rapport en pourcentage du nombre de points de chaque classe par rapport au nombre total de points, correspondant à la surface totale de l'échantillon 1.

Ainsi, il est possible d'obtenir automatiquement la proportion de tissus possédant une fibrose pathologique par rapport aux autres tissus sains. Dès lors, il n'est plus nécessaire à un opérateur spécialisé d'interpréter les résultats, qui sont directement disponibles sous forme d'une valeur représentative de l'avancement de la fibrose.

De façon subsidiaire, l'invention prévoit d'améliorer la pertinence du résultat 10 issu du traitement automatique, en combinant tous les résultats au sein d'une base de données. Dès lors, le résultat obtenu vient se coupler aux résultats établis précédemment lors de l'analyse antérieure d'autres échantillons. Pour ce faire, on combine ledit résultat 10 audit modèle 7 avant de réitérer ledit procédé avec un autre échantillon. Cette évolutivité du modèle permet en particulier d'affiner les classes spectrales afin d'inclure ou d'exclure des pixels dans une classe donnée et par là même améliorer la robustesse du modèle

Comme évoqué précédemment, l'invention a pour objet un procédé de détection et de quantification de la fibrose, dans lequel au moins :
- on positionne sur un support 2 un échantillon tissulaire 1 fixé non coloré ;
- on effectue une acquisition infrarouge par balayage de points dudit échantillon 1 ;
- on convertit le spectre infrarouge rendu par chaque point acquis en au moins une image spectrale 6 ;
- on effectue un traitement numérique de ladite image spectrale 6 par discrimination entre au moins le collagène physiologique et le collagène pathologique issue de la fibrose en attribuant à chaque point une classe spectrale par comparaison statistique avec un modèle numérique de spectres ;
- on quantifie les points possédant une classe spectrale similaire et on enregistre le résultat.

L'invention concerne aussi un dispositif de détection et de quantification de la fibrose, comprenant au moins :
- des moyens de réception 3 d'un support 2 d'un échantillon tissulaire 1 ;
- des moyens d'acquisition 4 par spectroscopie infrarouge à transformation de Fournier positionnés en vis-â-vis desdits moyens de réception 3, lesdits moyens d'acquisition 4 convertissant le spectre infrarouge rendu par chaque point acquis en au moins une image spectrale 6 ;
- des moyens de traitement numérique de ladite image spectrale 6 par discrimination entre au moins le collagène physiologique et le collagène pathologique en attribuant à chaque point une classe spectrale par comparaison statistique avec un modèle numérique de spectres dont est pourvu lesdits moyens de traitement, lesdits moyens de traitement quantifiant les points possédant une classe spectrale similaire ;
- des moyens d'enregistrement numérique du résultat desdits moyens de traitement.

De façon subsidiaire, tous les éléments, les aspects et les moyens numériques et informatiques, en particulier les algorithmes et les modèles numériques, sont exécutés au travers des terminaux numériques et informatiques dédiés, notamment connectés entre eux par l'intermédiaire de réseaux et de moyens de communication adaptés.

## Revendications

1. Procédé de détection et de quantification de la fibrose, dans lequel au moins :
- on positionne sur un support un échantillon tissulaire fixé non coloré ;
- on effectue une acquisition infrarouge par balayage de points dudit échantillon ;
- on convertit le spectre infrarouge rendu par chaque point acquis en au moins une image spectrale ;
- on effectue un traitement numérique de ladite image spectrale par discrimination entre au moins le collagène physiologique et le collagène pathologique issu de la fibrose en attribuant à chaque point une classe spectrale définie par un algorithme classant les spectres selon leur similitudes spectrale ;
- puis on attribue à chaque classe un état par comparaison statistique avec un modèle numérique de spectres ;
- on quantifie la surface relative occupée par les points de chaque état et on enregistre le résultat.

2. Procédé selon la revendication 1, **caractérisé par le fait que** ladite acquisition infrarouge s'effectue par spectroscopie infrarouge à transformation de Fourrier.

3. Procédé selon l'une quelconque des revendications précédentes, **caractérisé en ce que** ledit traitement comprend une étape préalable de prétraitement, consistant en l'élimination spectrale des bruits de fond provenant du support et du détecteur et en la neutralisation des interférences de la paraffine.

4. Procédé selon l'une quelconque des revendications précédentes, **caractérisé en ce que** lesdites classes sont au nombre de quatre, dont les états sont nommés : normal, fibrose, collagène non fibreux, inflammation.

5. Procédé selon l'une quelconque des revendications précédentes, **caractérisé en ce que** ladite discrimination s'effectue par comparaison de la valeur du spectre de chaque point avec des plages de valeurs de spectres dudit modèle,

6. Procédé selon l'une quelconque des revendications précédentes, **caractérisé en ce qu'**on quantifie statistiquement les points d'une même classe sous la forme d'un pourcentage surfacique de la surface totale dudit échantillon.

7. Procédé selon l'une quelconque des revendications précédentes, **caractérisé en ce qu'**on combine ledit résultat audit modèle avant de réitérer ledit procédé avec un autre échantillon.

8. Dispositif de détection et de quantification de la fibrose, comprenant au moins :
- des moyens de réception d'un support d'un échantillon tissulaire ;
- des moyens d'acquisition par spectroscopie infrarouge à transformation de Fournier positionnés en vis-à-vis desdits moyens de réception, lesdits moyens d'acquisition convertissant le spectre infrarouge rendu par chaque point acquis en au moins une image spectrale ;
- des moyens de traitement numérique de ladite image spectrale par discrimination entre au moins le collagène physiologique et le collagène pathologique en attribuant à chaque point une classe spectrale par comparaison statistique avec un modèle numérique de spectres dont est pourvu lesdits moyens de traitement, lesdits moyens de traitement quantifiant les points possédant une classe spectrale similaire ;
- des moyens d'enregistrement numérique du résultat desdits moyens de traitement.

9. Dispositif selon la revendication précédente, **caractérise par le fait que** lesdits moyens de réception sont motorisés et que ledit dispositif comprend des moyens de contrôle des déplacements desdits moyens de réception, de manière à effectuer un balayage relatif dudit support par lesdits moyens d'acquisition infrarouge.

## Patentansprüche

1. Verfahren zum Nachweis und zur Quantifizierung von Fibrose, wobei mindestens :
- eine ungefärbte befestigte Gewebeprobe auf einen Träger positioniert wird;
- eine Infraroterfassung durch Abtasten von Punkten der Probe durchgeführt wird;
- das von jedem erfassten Punkt wiedergegebene Infrarotspektrum in mindestens ein Spektralbild umgewandelt wird ;
- eine digitale Verarbeitung des Spektralbildes durch Diskriminierung zwischen zumindest dem physiologischen Kollagen und dem aus der Fibrose resultierenden pathologischen Kollagen dadurch durchgeführt wird, dass jedem Punkt eine Spektralklasse zugeordnet wird, die durch einen Algorithmus definiert wird, der die Spektren nach ihrer spektralen Ähnlichkeit klassifiziert;
- danach jeder Klasse ein Zustand durch statistischen Vergleich mit einem numerischen Spektrenmodell zugewiesen wird;
- die von den Punkten jedes Zustands eingenomme relative Oberfläche quantifiziert wird, und das Ergebnis aufgezeichnet wird.

2. Verfahren nach Anspruch 1, **dadurch gekennzeichnet, dass** die Infraroterfassung mittels Fourier-Transformation-Infrarotspektroskopie durchgeführt wird.

3. Verfahren nach irgendeinem der vorhergehenden Ansprüche, **dadurch gekennzeichnet, dass** die Behandlung einen vorabgehenden Vorbehandlungsschritt umfasst, der darin besteht, die von dem Träger und dem Detektor stammenden Hintergrundgeräusche spektral zu entfernen und die Interferenzen des Paraffins zu neutralisieren.

4. Verfahren nach irgendeinem der vorhergehenden Ansprüche, **dadurch gekennzeichnet, dass** die Klassen vier an der Zahl sind, deren Zustände wie folgt bezeichnet sind: normal, Fibrose, nicht-faseriges Kollagen, Entzündung.

5. Verfahren nach irgendeinem der vorhergehenden Ansprüche, **dadurch gekennzeichnet, dass** die besagte Diskriminierung dadurch durchgeführt wird, dass der Wert des Spektrums jedes Punkts mit Bereichen von Spektralwerten des besagten Modells verglichen wird.

6. Verfahren nach irgendeinem der vorhergehenden Ansprüche, **dadurch gekennzeichnet, dass** die Punkte einer gleichen Klasse statistisch als ein Oberflächenprozentsatz der Gesamtoberfläche der Probe quantisiert werden.

7. Verfahren nach irgendeinem der vorhergehenden Ansprüche, **dadurch gekennzeichnet, dass** das besagte Ergebnis mit dem Modell kombiniert wird, bevor das Verfahren mit einer anderen Probe wiederholt wird.

8. Vorrichtung zum Nachweis und zur Quantifizierung von Fibrose, umfassend mindestens :
- Mittel zum Aufnehmen eines Trägers einer Gewebeprobe;
- Mittel zum Erfassen mittels Fourier-Transformations-Infrarotspektroskopie, die gegenüber den besagten Aufnahmemitteln positioniert sind, wobei die besagten Erfassungsmittel das von jedem erfassten Punkt wiedergegebene Infrarotspektrum in mindestens ein Spektralbild umwandeln;
- Mittel zum digitalen Verarbeiten des Spektralbildes durch Diskriminierung zwischen zumindest dem physiologischen Kollagen und dem pathologischen Kollagen dadurch, dass jedem Punkt eine Spektralklasse durch statistischen Vergleich mit einem numerischen Modell von Spektren, mit dem die besagten Verarbeitungsmittel versehen sind, zugeordnet wird, wobei die besagten Verarbeitungsmittel die Punkte quantifizieren, die eine ähnliche Spektralklasse besitzen;
- Mittel zum digitalen Aufzeichnen des Ergebnisses der besagten Verarbeitungsmittel.

9. Vorrichtung nach dem vorhergehenden Anspruch, **dadurch gekennzeichnet, dass** die besagten Aufnahmemittel motorisiert sind und dass die besagte Vorrichtung Mittel zur Steuerung der Bewegungen der besagten Aufnahmemittel umfasst, um so eine relative Abtastung des besagten Trägers mittels der besagten Infrarot-Erfassungsmittel durchzuführen.

## Claims

1. A method for detecting and quantifying fibrosis, wherein at least:
- a non-coloured fixed tissue sample is positioned on a support;
- an infrared acquisition is performed by scanning points of said sample;
- the infrared spectrum rendered by each acquired point is converted into at least one spectral image;
- a digital processing of said spectral image is performed by discriminating between at least the physiological collagen and the pathological collagen resulting from the fibrosis by assigning to each point a spectral class defined by an algorithm classifying the spectra according to their spectral similarities;
- then, to each class is assigned a state by statistical comparison with a numerical model of spectra;
- the relative area occupied by the points of each state is quantified and the result is recorded.

2. The method according to claim 1, wherein said infrared acquisition is performed by Fourier transform infrared spectroscopy.

3. The method according to any one of the preceding claims, wherein said treatment comprises a preliminary pretreatment step consisting in spectrally eliminating the background noises coming from the support and from the detector and in neutralizing the interferences of the paraffin.

4. The method according to any one of the preceding claims, wherein said classes are four in number, the states of which are designated as : normal, fibrosis, non-fibrous collagen, inflammation.

5. The method according to any one of the preceding claims, wherein said discrimination is performed by comparing the value of the spectrum of each point with ranges of spectral values of said model.

6. The method according to any one of the preceding claims, wherein the points of the same class are statistically quantified in the form of a surface percentage of the total surface of said sample.

7. The method according to any one of the preceding claims, wherein said result is combined with said model before reiterating said method with another sample.

8. A device for detecting and quantifying fibrosis, comprising at least:
- means for receiving a support of a tissue sample;
- means for acquiring by Fourier transform infrared spectroscopy positioned in front of said receiving means, said acquisition means converting the infrared spectrum rendered by each acquired point into at least one spectral image;
- means for digitally processing said spectral image by discriminating between at least the physiological collagen and the pathological collagen by assigning to each point a spectral class by statistical comparison with a numerical model of spectra said processing means are provided with, said processing means quantizing the points having a similar spectral class;
- means for digitally recording the result of said processing means.

9. Device according to the preceding claim, wherein said receiving means are motorized and said device comprises means for controlling the movements of said receiving means, so as to perform a relative scanning of said medium by means of said infrared acquisition means.
